Europäisches Patentamt

European Patent Office

Office européen des brevets

(19)

(11) **EP 1 516 914 A1**

(12) **EUROPEAN PATENT APPLICATION**
published in accordance with Art. 158(3) EPC

(43) Date of publication:
**23.03.2005 Bulletin 2005/12**

(21) Application number: **03705054.9**

(22) Date of filing: **07.02.2003**

(51) Int Cl.[7]: **C11D 1/28**, C11D 1/74,
C11D 3/04, C11D 3/20,
C11D 3/37, C11D 3/33,
C11D 17/00, A61K 7/00,
A61K 7/075, A61K 7/50

(86) International application number:
**PCT/JP2003/001298**

(87) International publication number:
**WO 2004/000982 (31.12.2003 Gazette 2004/01)**

(84) Designated Contracting States:
**AT BE BG CH CY CZ DE DK EE ES FI FR GB GR
HU IE IT LI LU MC NL PT SE SI SK TR**

(30) Priority: **25.06.2002 JP 2002184157
02.08.2002 JP 2002226438**

(71) Applicant: **SHISEIDO COMPANY LIMITED
Chuo-ku, Tokyo 104-8010 (JP)**

(72) Inventors:
 • **KINOSHITA, Koichi,
 c/o SHISEIDO RESEARCH CENTER
 Yokohama-shi, Kanagawa 224-8558 (JP)**
 • **NODA, Akira,
 c/o SHISEIDO RESEARCH CENTER
 Yokohama-shi, Kanagawa 224-8558 (JP)**

 • **FUKUDA, Toshio,
 c/o SHISEIDO RESEARCH CENTER
 Yokohama-shi, Kanagawa 224-8558 (JP)**
 • **NAKAMA, Yasunari,
 c/o SHISEIDO RESEARCH CENTER
 Yokohama-shi, Kanagawa 224-8558 (JP)**
 • **KIMURA, Tomohiko,
 c/o SHISEIDO RESEARCH CENTER
 Yokohama-shi, Kanagawa 224-8558 (JP)**

(74) Representative: **Merkle, Gebhard
TER MEER STEINMEISTER & PARTNER GbR,
Patentanwälte,
Mauerkircherstrasse 45
81679 München (DE)**

(54) **DETERGENT COMPOSITIONS**

(57) The present invention provides a paste or solid cleaning agent composition for cleaning human skin, hair and such that gives a mild and refreshing tactile sensation, and exhibits superior stability and a sufficient foaming performance. The first portion of the present invention characteristically comprises (a) 5-50 mass % of a specific acyl salt anionic surfactant, (b) one, two or more chosen from inorganic salts, organic acids, and organic salts, (c) polyethylene glycol, and (d) water wherein the total electrolyte molar concentration is 1.8 mol/kg or more. The second portion of the present invention characteristically is in a paste or solid form and has a system melting point of 40°C or higher, comprising (a) an anionic surfactant having a Kraft point of 40°C or lower, (b) one, two, or more chosen from inorganic salts and organic salts, (c) trihydric or higher polyol, and (d) water.

EP 1 516 914 A1

**Description**

TECHNICAL FIELD

The present invention relates to a paste or solid cleaning agent composition.

The first portion of the present invention relates to a paste or solid cleaning agent composition for cleaning human skin, hair and such that gives a mild and refreshing tactile sensation, and exhibits superior stability above 45°C and sufficient foaming performance (A: claims 1-7).

The second portion of the present invention relates to a paste or solid cleaning agent composition for cleaning human skin, hair and such (claims 8-14). More specifically, the present invention relates to a paste or solid cleaning agent composition that gives a refreshing tactile sensation, and exhibits superior stability and sufficient foaming performance (claims 8-11). The present invention also relates to a paste or solid cleaning agent composition that gives a refreshing tactile sensation, exhibits stability and sufficient foaming performance, does not give a tingling sensation at the time of use, and provides creamy foam (claims 12-14).

BACKGROUND ART

A cleaning agent composition usually contains as a main ingredient an anionic surfactant because it is superior in terms of the foaming performance and cleaning power. For such a cleaning agent composition to assume a paste form, the anionic surfactant used needs to have a high Kraft point (the temperature at which the surfactant's hydrated crystals dissolve in water) and be in a state of hydrated crystals at ordinary temperatures. The carbon chains on the hydrophobic base portion of an anionic surfactant can be made longer to increase the Kraft point and give a paste-like appearance to the cleaning agent composition; this, however, significantly reduces the foaming performance, which is an essential function of the cleaning agent.

Examples of anionic surfactants that exhibit good foaming and sufficient cleaning power include those whose alkyl chain length is 12 carbons (lauryl) and 14 (myristyl). Of the anionic surfactants having the alkyl chain length as described above, examples of those that assume a paste form at ordinary temperatures include fatty acid salts (soap), acylglutamates, acylglycine salts, isethionates, and alkylsulfosuccinates; they are very few compared with the total number of anionic surfactants.

Many anionic surfactants having a alkyl chain length of 12 or 14 carbons have a Kraft point lower than ordinary temperatures and it is not possible to prepare a paste-like cleaning agent composition by using them as they are. A paste form can be forcibly achieved by adding a solidifier such as ethylene glycol distearate, but in this case foaming is reduced; also, if the hydrophilic base portion is a group other than a carboxyl group, such as a sulfate group or sulfonate group, there is a problem in that the sensation during use is not adequate due to excessive sliminess during rinsing, which is hard to rinse off.

Some of commercial paste base agents are called the fatty acid soap type, which are prepared by partially neutralizing the higher fatty acid with Na or K. However, the fatty acid soap type face washing foam does not provide smooth rinsing and also significantly lacks in terms of a moist and smooth sensation after drying.

In order to achieve a moist sensation after drying, there have been some face washing foam commercial products adjusted to a weakly acidic pH by using acylglutamates (Japanese Patent Publication No. S61-10445 bulletin, Japanese Patent Laid-Open No. 2002-20786 bulletin), monoalkylphosphates (Japanese Patent Laid-Open No. H11-189786 bulletin, Japanese Patent Laid-Open No. 2000-178173 bulletin), alkali metal salt of acylglycines (Japanese Patent Laid-Open No. H2002-20267 bulletin), etc. in addition to the fatty acid soap type. Fatty acid soaps cannot be made weakly acidic due to a stability problem; on the other hand, these base agents have the advantage of allowing a range of pH from weakly acidic to alkaline. However, they do not reach a satisfactory level in terms of smoothness during rinsing and moist sensation and smoothness after drying; also, depending on the base agent, some problems are not resolved yet, such as lacking hardness leading to dripping, poor stability of the external appearance at higher temperatures, and excessive hardness at lower temperatures.

Blending a small amount of acylmethyl taurine salt in the aforementioned base agent has been tried. However, in order to maintain the paste form or to maintain the high temperature stability, the maximum blend ratio of the acylmethyl taurine salt is limited to about 4 mass %.

DISCLOSURE OF INVENTION

[A: Invention defined in claims 1-7]

The object of the first portion of the present invention is to solve the aforementioned problems and provide a cleaning agent composition that assumes a paste or solid form in a wide temperature range, particularly at high temperatures, foams adequately, gives a good sensation during use without causing a tingling sensation of the skin, and exhibits superior stability.

In order to achieve the aforementioned object, the present invention provides a paste or solid cleaning agent composition having a total electrolyte molar concentration of 1.8 mol/kg or more, comprising (a) an acyl salt type anionic surfactant represented by the following general formula (1), (b) one, two, or more chosen from inorganic salts and organic salts, (c) polyethylene glycol, and (d) water.

$$R^1CO\text{-}NR^2CH_2CH_2SO_3X \tag{1}$$

(In this formula, $R^1$ denotes a hydrocarbon group having 10-24 carbon atoms; $R^2$ denotes a hydrogen atom or methyl group; and X denotes an alkali metal, alkali earth metal, ammonium, or organic amine.)

[B: Invention defined in claims 8-14]

The object of the second portion of the present invention is to solve the aforementioned problems and provide a cleaning agent composition that assumes a paste or solid form in a wide temperature range, foams adequately, gives a good sensation during use without causing sliminess during rinsing, and exhibits superior stability. Another object of the present invention is to provide a cleaning agent composition that, in addition to having the aforementioned effects, gives no tingling sensation during use and exhibits a creamy foam quality.

In order to achieve the aforementioned object, the present invention provides a paste or solid cleaning agent composition having a melting point of 40°C or higher, comprising (a) an anionic surfactant having a Kraft point of 40°C or lower, (b) one, two, or more chosen from inorganic salts and organic salts, (c) trihydric or higher polyol, and (d) water.

Also, the present invention provides the aforementioned cleaning agent composition wherein ingredient (a) is one, two, or more chosen from long chain acyl taurine salts represented by the following general formula (I).

$$R_1CO\text{-}NR_2\text{-}CH_2CH_2SO_3M \tag{I}$$

(In this formula, $R_1$ denotes a saturated or unsaturated hydrocarbon group with an average number of carbon atoms of 7-19; $R_2$ denotes an alkyl group with an average number of carbon atoms of 1-3; and M denotes an alkali metal, alkali earth metal, ammonium, or organic amine or derivative.)

The present invention also provides the aforementioned cleaning agent composition wherein at least one of the constituents of the (b) ingredient is the same type of metal ion salt as the counter ion of the (a) ingredient.

Also, the present invention provides the aforementioned cleaning agent composition wherein the (b) ingredient contains the same type of metal ion salt as the counter ion of the (a) ingredient and its blend ratio (molar ratio) is one or more compared with the metal ion salt of types other than the counter ion of the (a) ingredient.

Also, the present invention provides the aforementioned cleaning agent composition comprising, in addition to the (a)-(d) ingredients, (e) one, two, or more chosen from taurines and nonionic surfactants having a HLB of 10 or more.

Also, the present invention provides the aforementioned cleaning agent composition wherein the nonionic surfactant having a HLB of 10 or more that is used as the (e) ingredient is one, two, or more chosen from POE (= polyoxyethylene) glycerin monostearate, POE dialkylether, POE hydrogenated castor oil, and its/their derivatives.

Also, the present invention provides the aforementioned cleaning agent composition wherein the melting point is 45°C or higher for the system containing the aforementioned (a)-(e) ingredients.

BEST MODE FOR CARRYING OUT THE INVENTION

[A: Invention defined in claims 1-7]

The first portion of the present invention is described in detail below.

In the present invention, the (a) ingredient is represented by the aforementioned general formula (1). Examples of $R^1$ include residues of fatty acids such as lauric acid, myristic acid, and stearic acid; preferably lauric acid and myristic acid. Particularly preferable are coconut fatty acid and palm fatty acid. Examples of the salt include alkali metals (for example, lithium, potassium, sodium, etc.), alkali earth metals (for example, calcium, magnesium, etc.), ammonium, and organic amine (for example, monoethanolamine, diethanolamine, triethanolamine, etc.).

Particularly preferable in the present invention are sodium N-methyl cocoyl taurate represented by the following general formula (2) or sodium cocoyl taurate represented by the following general formula (3).

$$R^3CO\text{-}N(CH_3)CH_2CH_2SO_3^-\cdot Na \tag{2}$$

$$R^3CO\text{-}NHCH_2CH_2SO_3^-\cdot Na \tag{3}$$

(In this formula, $R^3CO$ denotes a coconut fatty acid residue.)

The blend ratio of the (a) ingredient is preferably 5-50 mass %, and more preferably 10-30 mass %. If the blend ratio is too small, then foaming becomes poor; if the blend ratio is too large, then there are shortcomings such as reduced productivity and difficulty in taking the product out of the container due to hardness.

Examples of preferably used inorganic salts for the (b) ingredient include chlorides, sulfates, bromides, etc. of sodium, potassium, magnesium, and calcium. Preferably used organic salts include sodium salts, potassium salts, magnesium salts, calcium salts, etc. of citric acid, succinic acid, lactic acid, malic acid, glycolic acid, tartaric acid, or acidic amino acids such as glutamic acid and aspartic acid. Of these, particularly preferable are sodium chloride and citrates.

For the (b) ingredient, one, two or more of these can be used; at least one constituent of the (b) ingredient should preferably be the same type of the metal ion salt as the counter ion in the aforementioned (a) ingredient. The molar ratio of the same type of metal ion salt as the counter ion of the (a) ingredient should preferably be one or more compared with the metal ion salt of types other than the counter ion of the (a) ingredient. If the molar ratio of "the same type of the metal ion salt as the counter ion of the (a) ingredient / the metal ion salt of types other than the counter ion of the (a) ingredient" is less than one, then, due to the eutectic point phenomenon, the Kraft point of the (a) ingredient may not increase enough and the stability may become poor.

The blend ratio of the (b) ingredient is not limited in particular as long as the melting point of the composition can be 45°C or higher in the presence of the other ingredients; the preferable range is approximately 1-10 mass %. If the (b) ingredient is too little then the paste form becomes harder to maintain; if the (b) ingredient is too much then a tingling sensation may arise during use. As mentioned later, the blend ratio of the (b) ingredient can be reduced by using polyethylene glycol for the polyol.

Polyethylene glycol, which is the (c) ingredient, is added to maintain a more stable paste or solid form at high temperatures. Of these, particularly preferable are polyethylene glycols having a molecular weight of 200-1,000. When polyethylene glycol is used, the paste form can be maintained even when the blend ratio of the salt is reduced down to approximately 5 mass %.

The blend ratio of (c) polyethylene glycol is preferably 2-40 mass % of the cleaning agent composition of the present invention. If there is too much of (c) polyethylene glycol, then the refreshing sensation at the time of rinsing is deficient; if there is too little, then the high temperature stability is deficient and the moist sensation is also deficient.

The blend ratio of water, the (d) ingredient, can be approximately 5-60 mass % in the cleaning agent composition of the present invention.

The cleaning agent composition of the present invention contains the aforementioned (a)-(d) ingredients as essential ingredients, has a melting point of 40°C or higher, and assumes a paste or solid form. In the present invention, the paste form refers to a state in which hydrated crystals of the (a) ingredient precipitate; therefore the external appearance is turbid and white and the hardness measured by determining the yielding point using a Vickers hardness tester at 25°C is 2 or more and 50 or less.

In the cleaning agent composition of the present invention, the addition of the (b)-(d) ingredients to the (a) ingredient gradually makes it harder for the (a) ingredient in the composition system to dissolve and the melting point of the final composition system becomes higher than 45°C ; this is mainly due to the salting out effect caused by the (b) ingredient and changes in the hydrogen bond state of water caused by the (c) ingredient.

In the present invention, it is preferable to add (e) nonionic surfactant in addition to the aforementioned essential ingredients. Examples of such (e) nonionic surfactant include higher fatty acid esters of polyethylene glycol, polyoxyethylenesorbitan fatty acid esters, polyoxyethylene sucrose fatty acid esters, ethylene oxide derivatives of glycerin fatty acid esters, ethylene oxide derivatives of propylene glycol fatty acid esters, polyoxyethylene alkyl ether, polyoxyethylene/polyoxypropylene block copolymers, polyoxyethylene alkylphenyl ethers, polyoxyethylene hydrogenated castor oil, higher fatty acid esters of polyglycerin, alkanolamides, polyoxyethylene fatty acid amides, and polyoxypropylene fatty acid amides; nonionic surfactants having a HLB of 10-15 are preferable, and particularly preferable are polyoxyethylene glycerin monoisostearates having a HLB of 10-15. Addition of (e) nonionic surfactant prevents the tingling sensation on the skin during use and provides good usability; foam quality and foam quantity are also improved.

The blend ratio of the (e) ingredient is preferably approximately 0.01-5 mass % of the cleaning agent composition of the present invention. If there is too much (e) nonionic surfactant, then the refreshing sensation at the time of rinsing is deficient.

In the present invention, it is preferable to add (f) one, two or more chosen from taurine, N-methyltaurine, and N,N-dimethyltaurine because this addition further reduces the tingling sensation on the skin during use and also contributes to achieving a paste form.

The electrolyte molar concentration of the cleaning agent composition of the present invention is 1.8 mol/kg or more. If it is less than 1.8 mol/kg, then the high temperature stability becomes deficient, which is not preferable.

The (b) ingredient is required for achieving the paste form but also causes a tingling sensation; therefore its total blend ratio should be 5 mass % or less. However, if it is less than 5 mass %, then the electrolyte molar concentration is low and the electrolyte molar concentration required to achieve the paste form is difficult to reach. For example, 5 mass % is 1. 7 mol/kg for sodium chloride. Addition of the (f) ingredient not only prevents the tingling sensation even when the concentration of the (b) ingredient reaches approximately 8 mass % but also contributes to reaching the electrolyte molar concentration required for achieving the paste form, thus allowing both achieving the paste form and preventing the tingling sensation. Addition of the (e) ingredient does not contribute to the electrolyte molar concentration but reduces the tingling sensation, making it easy to increase the salt concentration in order to achieve the required electrolyte molar concentration.

In addition to the aforementioned ingredients, ingredients that usually can be added to a cleaning agent composition may be added as necessary in an appropriate amount; examples include polymers such as cationic polymers, anionic polymers, ampholytic polymers, and nonionic polymers, as well as humectants, perfumes, preservatives, colorings, antioxidants, beautifying ingredients, and powders. In the present invention, mica, kaolin, silica, talc, sericite, montmorillonite, zeolite, polyethylene, nylon, poly methyl methacrylate, or cellulose can be added as the powder ingredient to improve the usability. Oil components that are liquid or solid at ordinary temperatures can be added to further reduce irritation without substantial deterioration of the high temperature stability; examples of such oil components include silicone derivatives, higher alcohols, higher fatty acids, higher fatty acid esters of higher alcohols, alkyl ethers of higher alcohols, fatty acid esters of glycerin, fatty acid esters of ethylene glycol, and fatty acid esters of pentaerythritol.

[B: Invention defined in claims 8-14]

The second portion of the present invention is described in detail below.

The (a) ingredient is an anionic surfactant having a Kraft point of 40°C or lower. Selection of such an anionic surfactant is not limited in particular as long as it is commonly used in cleaning agents; examples include fatty acid salts, α-acylsulfonates, alkylsulfonates, alkylallyl- and alkylnaphthalene- sulfonates, alkylsulfates, polyoxyethylene alkyl ether sulfates, alkylamide sulfates, alkylphosphates, alkylamide phosphates, N-long chain acylamino acid salts, alkyl ether carboxylates, alkylhydroxy ether carboxylates, and long chain acyltaurine salts represented by the following general formula (I)

$$R_1CO\text{-}NR_2\text{-}CH_2CH_2SO_3M \qquad\qquad\qquad (I)$$

(In this formula, $R_1$ denotes a saturated or unsaturated hydrocarbon group with an average number of carbon atoms of 7-19; $R_2$ denotes an alkyl group with an average number of carbon atoms of 1-3; and M denotes an alkali metal, alkali earth metal, ammonium, or organic amine or derivative).

Of these, the long chain acyltaurine salts represented by the aforementioned general formula (I), polyoxyethylene alkyl ether sulfates, etc. are preferable, and the long chain acyltaurine salts represented by the aforementioned general formula (I) are particularly preferable. Examples of the long chain acyltaurine salts represented by the aforementioned general formula (I) that are used in the present invention include lauroylmethyltaurine salts, myristoylmethyltaurine salts, cocoyl methyltaurine salts, lauroylethyltaurine salts, myristoylethyltaurine salts, cocoyl ethyltaurine salts, lauroyltaurine salts, and cocoyl taurine salts; needless to say, selection is not limited to these examples.

Examples of the counter ion for the (a) ingredient include alkali metals (such as lithium, potassium, and sodium), alkali earth metals (such as calcium and magnesium), organic amines (such as monoethanolamine, diethanolamine, and triethanolamine), and ammonium. Of these, sodium and potassium are preferable.

From the point of view of foaming performance, the (a) ingredient should preferably be those having 12 or 14 carbon atoms in the alkyl group or acyl group in the main chain; but selection is not limited to these. When a fatty acid salt is used for the (a) ingredient, mixing in a calcium salt as the (b) ingredient (mentioned later) will result in formation of a nonsoluble substance; therefore joint use of a sodium salt and a potassium salt is preferable. For the (a) ingredient, one, two or more types can be used.

In order to maintain the paste/solid form of the cleaning agent composition, the blend ratio of the (a) ingredient should preferably be approximately 5 mass % or more of the cleaning agent composition of the present invention; particularly preferable is approximately 10 mass % or more.

For the inorganic salt used in the (b) ingredient, chlorides, sulfates, bromides, etc. of sodium, potassium, magnesium, and calcium are preferable. Preferably used organic salts include sodium salts, potassium salts, magnesium salts, calcium salts, etc. of citric acid, succinic acid, lactic acid, or acidic amino acids such as glutamic acid and aspartic acid.

For the (b) ingredient, one, two or more of these can be used; at least one constituent of the (b) ingredients should preferably be the same type of metal ion salt as the counter ion in the aforementioned (a) ingredient. For the (b) ingredient, the molar ratio of the same type of metal ion salt as the counter ion of the (a) ingredient should preferably be one or more compared with the metal ion salt of types other than the counter ion of the (a) ingredient. If the molar ratio of "the same type of the metal ion salt as the counter ion of the (a) ingredient / the metal ion salt of types other than the counter ion of the (a) ingredient" is less than one, then, due to the eutectic point phenomenon, the Kraft point of the (a) ingredient may not increase enough and the stability may become poor. In the present invention, sodium salts, potassium salts, etc. are preferable for the same type of metal ion salt as the counter ion of the (a) ingredient.

The blend ratio of the (b) ingredient is not limited in particular as long as the melting point of the composition can be 40°C or higher in the presence of the other ingredients; the preferable range is approximately 1-10 mass %. If the (b) ingredient is too much then a tingling sensation may arise during use.

For the (c) ingredient, a polyol that is trihydric or higher is used in place of water to maintain a more stable paste/solid form at higher temperatures. Examples of such preferably used polyols include polyhydric alcohols such as glycerin, sorbitol, xylitol, and erythritol, and sugars such as sucrose and trehalose. One, two or more of these can be used for the (c) ingredient.

The blend ratio of the (c) ingredient is preferably 2-40 mass % of the cleaning agent composition of the present invention.

The blend ratio of water, the (d) ingredient, can be approximately 5-60 mass % in the cleaning agent composition of the present invention.

The cleaning agent composition of the present invention contains the aforementioned (a)-(d) ingredients as essential ingredients, has a melting point of 40°C or higher, and assumes a paste or solid form. In the present invention, the paste form refers to a state in which hydrated crystals of the (a) ingredient precipitate; therefore the external appearance is turbid and white, and, as an indication of the hardness, the viscosity should preferably be 3,000 mPa·s (30°C, using a B-type viscometer) or more.

In the present invention, the addition of the (b)-(d) ingredients to the (a) ingredient having a Kraft point of 40°C or lower gradually makes it harder for the (a) ingredient in the composition system to dissolve and the melting point of the final composition system becomes higher than 40°C ; this is mainly due to the salting out effect caused by the (b) ingredient and changes in the hydrogen bond state of water caused by the (c) ingredient.

In this invention, in addition to the aforementioned (a)-(d) ingredients, the (e) ingredient, which is one, two or more chosen from taurines and nonionic surfactants having a HLB of 10 or more, may be blended in.

For the taurines, those represented by the following general formula (II)

$$N(R_3)(R_4)CH_2CH_2SO_3M \tag{II}$$

(in this formula, $R_3$ and $R_4$ denote, independently to each other, a hydrogen atom or an alkyl group having an average of 1-3 carbon atoms; M denotes an alkali metal, alkali earth metal, ammonium, or organic amine or derivatives) are used. Specific examples include taurine, methyltaurine, sodium methyltaurate, sodium dimethyltaurate, sodium ethyltaurate, and potassium methyltaurate.

For the nonionic surfactant, those having a HLB of 10 or more are used; in view of the external stability and foam quality, those having a HLB of 10-18 are particularly preferable. Selection of the nonionic surfactant used in the present invention is not limited in particular as long as the HLB is within the range described above; examples include POE (= polyoxyethylene) glycerin monoisostearates such as POE (15) glycerin monoisostearate (HLB = 12), POE (25) glycerin monoisostearate (HLB = 15), POE (30) glycerin monoisostearate (HLB = 18), POE (60) glycerin monoisostearate (HLB = 19), and POE (90) glycerin monoisostearate (HLB = 22); POE dialkyl ethers such as POE (25) octyldodecyl ether (HLB = 14), POE (16) octyldodecyl ether (HLB = 12), and POE (25) decyltetradecyl ether (HLB = 14); POE hydrogenated castor oils such as POE (60) hydrogenated castor oil (HLB = 14), POE (40) hydrogenated castor oil (HLB = 12), and POE (80) hydrogenated castor oil (HLB = 16), as well as their derivatives; selection is not limited to these examples.

The HLB is calculated by using the Kawakami formula represented by the following equation:

$$HLB = 7 + 11.7 \cdot \log(MW/M0)$$

In this equation, MW denotes the molecular weight of the hydrophilic basic portion and M0 denotes the molecular weight of the lipophilic basic portion.

The addition of the (e) ingredient to the (a)-(d) ingredients allows a reduction in the blend ratio of the (b) ingredient down to 7 mass % where it would be 10 mass % if a four ingredient system of the (a)-(d) ingredients was used; this addition has an effect on foaming, stability, and the refreshing sensation and reduces the tingling sensation during use, and thus a paste/solid cleaning agent composition having a melting point of 45°C or higher can be obtained.

Ingredients that usually can be added to a cleaning agent composition may be added as necessary in an appropriate amount; examples include polymers (such as water soluble polymers), humectants, perfumes, preservatives, colorings, antioxidants, beautifying ingredients, and powders. In particular, the addition of a water soluble polymer can further improve the foam quality and usability. Preferable examples of the water soluble polymer include, but are not limited to, cationized cellulose, cationized guar gum, cationized starch, cationized locust bean gum, polyquaternium-6, polyquaternium-7, polyquaternium-39, hydroxyethyl cellulose, keltrol, and carboxyvinyl polymers. Also, there is no restriction due to their average molecular weight or electric charge density. The blend ratio of the water soluble polymer is preferably approximately 2 mass % or less of the cleaning agent composition of the present invention.

The present invention is preferably used for skin cleaning agents (such as face cleaning agents and body shampoos) and hair cleaning agents (such as shampoos).

EXAMPLES

[A: Invention defined in claims 1-7]

The first portion of the present invention is described in detail below by referring to Examples, but the present invention is not limited to these Examples. The blend ratios are all in mass % units.

First, the testing method and evaluation method used in Examples are described below.

(1) Kraft point

A differential scanning calorimeter (DSC) was used to measure the endothermic peak of the sample for water when raising the temperature; the Kraft point was defined as the peak top.

(2) External stability after one week at 45°C

After the sample was prepared, a prescribed amount of it was put into a glass bottle and stored in a 45°C thermostatic bath for one week, after which the external appearance was evaluated based on the following criteria.

(Evaluation)

◎ : The whole sample is homogeneously white and turbid; no clear portion is observed.
○ : A clear portion is observed in less than 5% of the whole sample.
△ : A clear portion is observed in 5% or more and less than 40% of the whole sample.
× : A clear portion is observed in 40% or more of the whole sample.

(3) Tingling sensation during use

A panel of 20 specialists applied 1 g of the sample directly on the face and washed the face; the sensory evaluation of the tingling sensation at that time was conducted using the following criteria.

◎ : 18 or more reported there was no tingling sensation during use.
○ : 15 or more and less than 17 reported there was no tingling sensation during use.
△ : 12 or more and less than 14 reported there was no tingling sensation during use.
▲ : 9 or more and less than 11 reported there was no tingling sensation during use.

×: 8 or less reported there was no tingling sensation during use.

(4) Amount of foam

Each 1 g of the sample was added to 20 mL of an aqueous solution (30°C) containing 100 ppm of calcium chloride; a 100 mL colorimetric tube with a ground-in stopper was used to apply 60 seconds of shaking at 2 strokes/second, after which the amount of foam was measured and evaluated using the following criteria.

ⓞ : Very good foaming (the amount of foam is 80 mL or more)
○: Good foaming (the amount of foam is 70 mL or more and less than 80 mL)
∆ : Moderate foaming (the amount of foam is 60 mL or more and less than 70 mL)
▲: Somewhat poor foaming (the amount of foam is 40 mL or more and less than 60 mL)
×: Poor foaming (the amount of foam is less than 40 mL)

(5) Smoothness at the time of rinsing

A panel of 20 specialists used 1 g of the sample to wash their faces in a normal way; the sensory evaluation of the smoothness at the time of rinsing was conducted using the following criteria.

ⓞ : 18 or more reported smoothness at the time of rinsing.
○: 15 or more and less than 17 reported smoothness at the time of rinsing.
∆ : 12 or more and less than 14 reported smoothness at the time of rinsing.
▲: 9 or more and less than 11 reported smoothness at the time of rinsing.
× : 8 or less reported smoothness at the time of rinsing.

(6) Moist sensation after drying

A panel of 20 specialists used 1 g of the sample to wash their faces in a normal way; the sensory evaluation of the moist sensation after drying was conducted using the following criteria.

ⓞ : 18 or more reported a moist sensation after drying.
○: 15 or more and less than 17 reported a moist sensation after drying.
∆ : 12 or more and less than 14 reported a moist sensation after drying.
▲: 9 or more and less than 11 reported a moist sensation after drying.
× : 8 or less reported a moist sensation after drying.

(7) Smoothness after drying

A panel of 20 specialists used 1 g of the sample to wash their faces in a normal way; the sensory evaluation of the smoothness after drying was conducted using the following criteria.

ⓞ : 18 or more reported smoothness after drying.
○: 15 or more and less than 17 reported smoothness after drying.
∆ : 12 or more and less than 14 reported smoothness after drying.
▲: 9 or more and less than 11 reported smoothness after drying.
× : 8 or less reported smoothness after drying. (8) pH

The mother solution of the sample was measured with a pH meter.

(9) Foam quality

A panel of 20 specialists used 1 g of the sample to wash their faces in a normal way; the sensory evaluation of the foam quality
(creaminess) was conducted using the following criteria.

ⓞ : 18 or more reported creaminess.
○: 15 or more and less than 17 reported creaminess.
∆ : 12 or more and less than 14 reported creaminess.

▲ : 9 or more and less than 11 reported creaminess. × : 8 or less reported creaminess.

(10) Refreshing sensation at the time of rinsing

A panel of 20 specialists used 1 g of the sample to wash their faces in a normal way; the sensory evaluation of the refreshing sensation at the time of rinsing was conducted using the following criteria.

◎ : 18 or more reported a refreshing sensation at the time of rinsing.
○ : 15 or more and less than 17 reported a refreshing sensation at the time of rinsing.
Δ : 12 or more and less than 14 reported a refreshing sensation at the time of rinsing.
Δ : 9 or more and less than 11 reported a refreshing sensation at the time of rinsing.
× : 8 or less reported a refreshing sensation at the time of rinsing.

(11) Temperature dependence of the hardness (low temperatures)

After the sample was prepared, a prescribed amount of it was put into a glass bottle and stored in a 0°C thermostatic bath for three days, after which the hardness was measured by using a Vickers hardness tester and evaluated using the following criteria.

(Evaluation)

◎ : The hardness at 0°C is less than 35.
○ : The hardness at 0°C is 35 or more and less than 50.
Δ : The hardness at 0°C is 50 or more and less than 65.
▲ : The hardness at 0°C is 65 or more and less than 85.
× : The hardness at 0°C is more than 85.

Experimental examples 1-12

Cleaning agent compositions consisting of the compositions (mass %) shown in the following Table 1a were prepared and the Kraft point, the external appearance after one week at 45°C, and the tingling sensation during use were investigated using the aforementioned criteria. The results are shown in Table 1a.
The samples were prepared by stirring/dissolving the raw materials at 75°C (the powders used in some Examples were dispersed), and lowering the temperature at a constant rate down to 25°C. The following experimental examples were prepared in the same manner, as well.

Table 1a

| Experimental examples | 1 | 2 | 3 | 4 | 5 | 6 | 7 | 8 | 9 | 10 | 11 | 12 |
|---|---|---|---|---|---|---|---|---|---|---|---|---|
| Na cocoyl methyltaurate | 20.0 | 20.0 | 20.0 | 20.0 | 20.0 | 20.0 | 20.0 | 20.0 | 20.0 | 20.0 | 20.0 | 20.0 |
| Sorbitol | 20.0 | – | – | – | – | – | – | – | – | – | – | – |
| Glycerin | – | 20.0 | – | – | – | – | – | – | – | – | – | – |
| PEG200 | – | – | 20.0 | – | – | – | – | – | – | – | – | – |
| PEG300 | – | – | – | 20.0 | – | – | – | – | – | – | – | – |
| PEG400 | – | – | – | – | 20.0 | – | – | – | – | – | – | – |
| PEG1000 | – | – | – | – | – | 20.0 | – | – | – | – | – | – |
| PEG20000 | – | – | – | – | – | – | 20.0 | – | – | – | – | – |
| 1,3-butylene glycol | – | – | – | – | – | – | – | 20.0 | – | – | – | – |
| Dipropylene glycol | – | – | – | – | – | – | – | – | 20.0 | – | – | – |
| PPG200 | – | – | – | – | – | – | – | – | – | 20.0 | – | – |
| Na citrate | 0.6 | 0.6 | 0.6 | 0.6 | 0.6 | 0.6 | 0.6 | 0.6 | 0.6 | 0.6 | 0.6 | 0.4 |
| Citric acid | 0.4 | 0.4 | 0.4 | 0.4 | 0.4 | 0.4 | 0.4 | 0.4 | 0.4 | 0.4 | 0.4 | 0.6 |
| Sodium chloride | 5.5 | 5.5 | 5.5 | 5.5 | 5.5 | 5.5 | 5.5 | 5.5 | 5.5 | 5.5 | 5.5 | 12.5 |
| Ion-exchanged water | Balance | Balance | Balance | Balance | Balance | Balance | Balance | Balance | Balance | Balance | Balance | Balance |
| Kraft point | 48.6 | 50.8 | 52.8 | 53.1 | 53.1 | 52.0 | 47.8 | 44.6 | 42.1 | 36.0 | 35.6 | 53.2 |
| External appearance stability after 1W at 45°C | △ | △ | ○ | ○ | ○ | ○ | △ | × | × | × | × | ○ |
| Tingling sensation during use | △ | △ | △ | △ | △ | △ | △ | △ | △ | △ | ◎ | × |

(The amount of sodium chloride is the total of the amount contained in the activator raw material as a side product and the amount newly added. This applies to subsequent examples as well.)

(The amount of sodium chloride is the total of the amount contained in the activator raw material as a side product and the amount newly added. This applies to subsequent examples as well.)

Table 1a is a comparison of polyols; in this table, PEG is an acronym of polyethylene glycol and the numbers are molecular weights. PPG is an acronym of polypropylene glycol. When polyethylene glycol was used, the obtained cleaning agent composition had a high Kraft point, good external appearance stability after 1 week at 45°C, and a reduced tingling sensation during use.

Experimental examples 13-27

Cleaning agent compositions consisting of the compositions (mass %) shown in the following Tables 2a and 3a were prepared and the Kraft point, the external appearance after one week at 45°C, the tingling sensation during use, and the amount of foam were investigated using the aforementioned criteria. The results are shown in Tables 2a and 3a.

Table 2a

| Experimental examples | 13 | 14 | 15 | 16 | 17 | 18 | 19 | 20 | 21 | 22 | 23 |
|---|---|---|---|---|---|---|---|---|---|---|---|
| Na cocoyl methyltaurate | 20.0 | 20.0 | 20.0 | 20.0 | 20.0 | 20.0 | 20.0 | 20.0 | 20.0 | 20.0 | 20.0 |
| PEG300 | 20.0 | 20.0 | 20.0 | 20.0 | 20.0 | 20.0 | 20.0 | 20.0 | 20.0 | 20.0 | 20.0 |
| Na benzoate | 0.3 | 0.3 | 0.3 | 0.3 | 0.3 | 0.3 | 0.3 | 0.3 | 0.3 | 0.3 | 0.3 |
| Na citrate | 0.4 | 0.4 | 0.4 | 0.4 | 0.4 | 0.6 | 0.6 | 3.0 | 7.9 | - | - |
| Citric acid | 0.6 | 0.6 | 0.6 | 0.6 | 0.6 | 1.0 | 1.0 | 0.7 | 1.2 | - | - |
| Tartaric acid | - | - | - | - | - | - | - | - | - | 2.3 | 5.6 |
| Sodium hydroxide | - | - | - | - | - | - | - | - | - | 1.2 | 3.0 |
| Sodium chloride | 3.5 | 5.5 | 1.0 | 1.0 | 1.0 | 1.0 | 1.0 | 1.0 | 1.0 | 1.0 | 1.0 |
| Sodium sulfate | - | - | 6.0 | - | - | - | - | - | - | - | - |
| Taurine | - | - | - | 4.5 | 13.0 | - | - | - | - | - | - |
| Na N-methyltaurate | - | - | - | - | - | 8.1 | 24.2 | - | - | - | - |
| Ion-exchanged water | | | | | | | | | | | |
| pH | 5.9 | 5.9 | 5.9 | 5.9 | 5.9 | 5.9 | 5.9 | 5.9 | 5.9 | 5.9 | 5.9 |
| Molar concentration (including taurines) | 1. 34 | 2.03 | 1.75 | 0.84 | 1.52 | 1.05 | 1.96 | 0.83 | 1. 52 | 0.84 | 1. 51 |
| Kraft point | 45.1 | 53.1 | 50.0 | 35.6 | 40.3 | 45.5 | 60.8 | 39.2 | 47.4 | 39.2 | 47.4 |
| External appearance stability after 1W at 45°C | ✕ | ○ | △ | ✕ | ✕ | ✕ | ○ | ✕ | ✕ | ✕ | ✕ |
| Tingling sensation during use | ○ | △ | ▲ | ◎ | ◎ | ◎ | ◎ | ○ | ▲ | ○ | ▲ |

Table 3a

| Experimental examples | 24 | 25 | 26 | 27 |
|---|---|---|---|---|
| Na cocoyl methyltaurate | 20.0 | 20. 0 | 20. 0 | 20.0 |
| PEG300 | 20. 0 | 20. 0 | 20.0 | 20.0 |
| Na benzoate | 0.3 | 0.3 | 0.3 | 0.3 |
| Na citrate | 0.6 | 2.6 | 0.6 | 0.6 |
| Citric acid | 0.4 | 0.4 | 0.4 | 1.6 |
| Sodium chloride | 5.5 | 5.5 | 5.5 | 5.5 |
| Taurine | - | - | 2.0 | - |
| Na N-methyltaurate | - | - | - | 2.5 |
| Ion-exchanged water | Balance | Balance | Balance | Balance |
| pH | 5.8 | 6.2 | 5.8 | 5.8 |
| Molar concentration (including taurines) | 2.03 | 2.30 | 2.20 | 2.24 |
| Kraft point | 53.0 | 56.2 | 55.1 | 57.9 |
| External appearance stability after 1W at 45°C | ○ | ◎ | ◎ | ◎ |
| Tingling sensation during use | △ | △ | ○ | ○ |
| Amount of foam | △ | △ | ○ | ○ |

Tables 2a and 3a show a comparison of the salts. The molar concentration (including taurines) refers to the total equivalent concentration (mol/kg) of Na benzoate, Na citrate, citric acid, tartaric acid, sodium hydroxide, sodium chloride, sodium sulfate, taurine, and Na N-methyltaurate. Tables 2 and 3 show that the external appearance stability at 45°C is maintained when the molar concentration is 1.8 mol/kg or more. Experimental examples 26 and 27 show that a stable composition can be obtained by adding taurine or taurate even when the salt concentration is low.

Experimental examples 28-33

Cleaning agent compositions consisting of the compositions (mass %) shown in the following Table 4a were prepared and the pH, the external appearance after one week at 45°C, the amount of foam, and the quality of foam were investigated using the aforementioned criteria. The results are shown in Table 4a.

Table 4a

| Experimental exanptes | 28 | 29 | 30 | 31 | 32 | 33 |
|---|---|---|---|---|---|---|
| Na cocoyl methyltaurate | 20.0 | 20.0 | 20.0 | 20.0 | 20.0 | 20.0 |
| PEG300 | 20.0 | 20.0 | 20.0 | 20.0 | 20.0 | 20.0 |
| Na benzoate | 0.3 | 0.3 | 0.3 | 0.3 | 0.3 | 0.3 |
| Na citrate | - | 0.2 | 0.4 | 0.6 | 0.8 | 1.0 |
| Citric acid | 1.0 | 0.8 | 0.6 | 0.4 | 0.2 | - |
| Sodium chloride | 5.5 | 5.5 | 5.5 | 5.5 | 5.5 | 5.5 |
| Ion-exchanged water | Balance | Balance | Balance | Balance | Balance | Balance |
| pH | 4.8 | 5.0 | 5.5 | 5.9 | 6.6 | 7.8 |
| External appearance stability after 1W at 45°C | △ | ○ | ○ | ○ | ○ | ○ |
| Amount of foam | ○ | ○ | ○ | △ | △ | ▲ |
| Quality of foam | △ | △ | △ | △ | ▲ | ▲ |

Table 4a is mainly a comparison of the amount and quality of foam at various pHs. Table 4a shows that the amount and quality of foam are satisfactory at pH = 4.8-5.9.

Example 1, Comparative examples 1-4

Cleaning agent compositions consisting of the compositions (mass %) shown in the following Table 5a were prepared and the temperature dependence of the hardness (at low temperatures), the external appearance after one week at 45°C, the amount of foam, smoothness at the time of rinsing, the moist sensation after drying, and smoothness after drying were investigated using the aforementioned criteria. The results are shown in Table 5a.

Table 5a

| Examples | 1 | 2 | | | | |
|---|---|---|---|---|---|---|
| Comparative examples | | | 1 | 2 | 3 | 4 |
| Na cocoyl methyltaurate | 20.0 | - | 2.4 | - | 1. 0 | - |
| K cocoyl taurate | - | 15.0 | - | - | - | - |
| Lauric acid | - | - | 2.0 | - | 4.5 | - |
| Myristic acid | - | - | - | 2.0 | 10.0 | - |
| Stearic acid | - | - | - | - | 12.0 | - |
| Potassium hydroxide | - | - | - | - | 4.4 | - |
| Potassium laurylphosphate | - | - | - | - | - | 15.0 |

Table 5a   (continued)

| Examples | 1 | 2 | | | | |
|---|---|---|---|---|---|---|
| Comparative examples | | | 1 | 2 | 3 | 4 |
| Na coooyl acylglutamate | - | - | 20.0 | 20 | - | - |
| Na cocoyl arylglycine | - | - | - | 18.0 | - | - |
| Na lauryl glycol acetate | - | - | - | 1.0 | - | - |
| POE glycerin monoisotearate (20E0) | 2.0 | 1. 5 | - | - | - | - |
| POE glycerin monoisotearate (60E0) | - | - | 3.0 | - | - | - |
| Cocoyl amidepropylbetaine | - | - | - | 2.0 | - | 2.0 |
| Sorbitol | - | - | - | - | - | 20.0 |
| Glycerin | 5.0 | 5.0 | 3.0 | 30.0 | 25.0 | - |
| PEG400 | 15.0 | 15.0 | 25.0 | - | 5.0 | - |
| PEG6000 | - | - | 2.0 | - | - | - |
| Polyethylene powder | - | - | - | 1. 0 | - | - |
| Talc | - | - | - | - | - | 4.0 |
| Carboxyvinyl polymer | - | - | - | - | - | 1.5 |
| Potassium hydroxide | - | - | - | - | 4.4 | - |
| Na N-methyltaurate | 2.0 | 2.0 | - | - | 4.4 | - |
| Na citrate | - | - | 0.1 | - | - | - |
| Citric acid | 1.5 | 1.5 | 0.5 | 1.0 | - | - |
| Potassium chloride | - | 5.0 | - | - | - | - |
| Sodium chloride | 5.0 | - | - | - | - | - |
| Perfume | Appropriate amount | Appropriate amount | Appropriate amount | Appropriate amount | Appropriate amount | Appropriate amount |
| Ion-exchanged water | Balance | Balance | Balance | Balance | Balance | Balance |
| External appearance | Paste | Paste | Paste | Paste | Paste | Liquid |
| pH (mother solution) | 5.5 | 5.6 | 5.4 | 6.5 | 11.0 | 6.2 |
| Temperature dependence of the hardness (low temperatures) | ◎ | ◎ | ▲ | Δ | Δ | ○ |

Table 5a   (continued)

| Examples | 1 | 2 | | | | |
|---|---|---|---|---|---|---|
| Comparative examples | | | 1 | 2 | 3 | 4 |
| External appearance stability after 1W at 45°C | ◎ | ◎ | ◎ | ◎ | ○ | Δ |
| Usability Amount of foam | ○ | ◎ | ○ | ○ | ◎ | ○ |
| Smoothness at the time of rinsing | ◎ | ◎ | ▲ | ▲ | × | ▲ |
| Moist sensation after drying | ◎ | ◎ | ○ | ▲ | ▲ | ○ |
| Smoothness after drying | ◎ | ◎ | ○ | ○ | ▲ | ○ |

Table 5a compares stability and usability when using different surfactants. Table 5a shows that the cleaning agent composition containing the (a) ingredient of the present invention is superior in both stability and usability.
Recipe examples of the cleaning agent composition of the present invention are shown below.

| Recipe example 1 (body shampoo) | |
|---|---|
| Sodium myristate taurate | 20.0 mass % |
| Cocoyl amidepropyl betaine | 1.0 |
| PEG 300 | 20.0 |
| PEG 10000 | 5.0 |
| Sodium chloride | 5.0 |
| Na L-glutamate | 0.4 |
| L-glutamic acid | 0.8 |
| Taurine | 4.0 |
| EMALEX GWIS-160 (Glyceryl POE isostearate (60EO) from Nihon Emulsion) | 0.5 |
| Theodol E-2025 (Octyldodeceth-25) | 1. 0 |
| Behenic acid | 1.5 |
| 2-octyldodecanol | 0. 1 |
| Merquart 100 (40% aqueous solution of polyquaternium from Nalco) | 0. 3 |
| Silica (spherical, average particle size 2 micrometers) | 0.5 |
| Trehalose | 0.2 |
| Phenoxy ethanol | 0.1 |
| Perfume | 0. 1 |
| Ion-exchanged water | Balance |

| Recipe example 2 (Face cleaning agent) | |
|---|---|
| Sodium cocoyl taurate | 10.0 mass % |
| PEG 400 | 15.0 |
| Sorbitol | 8.0 |
| Sodium chloride | 3.0 |
| Na tartarate | 0. 6 |

(continued)

| Recipe example 2 (Face cleaning agent) | |
|---|---|
| Tartaric acid | 0.4 |
| Taurine | 4. 0 |
| EMALEX GWIS-120 | 2. 0 |
|     (Glyceryl POE isostearate (20EO) from Nihon Emulsion) | |
| Nikkol HCO-60 | 1.0 |
|     (POE hydrogenated castor oil (60EO) from Nihon Chemicals) | |
| Monoglyceride stearate | 0.5 |
| Merquart 550 | 1. 0 |
|     (10% aqueous solution of polyquaternium-7 from Nalco) | |
| Mica | 0. 5 |
| Methylparaben | 0. 1 |
| L-hydroxyproline | 0. 1 |
| Perfume | 0.1 |
| Ion-exchanged water | Balance |

| Recipe example 3 (hair shampoo) | |
|---|---|
| Potassium cocoyl taurate | 15.0 mass % |
| Sodium laurylglycol acetate | 0.5 |
| PEG 600 | 10. 0 |
| Glycerin | 10.0 |
| Potassium chloride | 4.0 |
| Na citrate | 0. 6 |
| Citric acid | 2.4 |
| Sodium N-methyltaurate | 3.0 |
| Pluaronic L-64 | 0. 5 |
|     (POE (25) POP (30) copolymer) | |
| Nikkol HCO-60 | 1.0 |
|     (POE hydrogenated castor oil (60EO) from Nihon Chemicals) | |
| 12-hydroxystearic acid | 0.4 |
| Polymer JR | 0.4 |
|     (Cationized cellulose from TOHO Chemical Industry) | |
| Jaguar C-13S (Cationized guar gum) | 0.4 |
| Dimethylsilicone | 0.2 |
|     (10,000 cs-silicone made into a particle size of 30 nm) | |
| Talc | 1. 0 |
| Zanthoxylum extract | 1.0 |
| Na benzoate | 0.5 |
| Perfume | 0.1 |
| Ion-exchanged water | Balance |

| Recipe example 4 (Face cleaning agent) | |
|---|---|
| Sodium cocoyl taurate | 10. 0 mass % |
| Sodium cocoyl isethionate | 10.0 |
| PEG 400 | 20.0 |
| Dipropylene glycol | 1.0 |
| Sodium chloride | 4.0 |
| Na malate | 0.6 |

(continued)

| Recipe example 4 (Face cleaning agent) | |
|---|---|
| Malic acid | 2.4 |
| Sodium N-methyltaurate | 3.0 |
| Nikkol HC0-60 | 1. 0 |
| (POE hydrogenated castor oil (60EO) from Nihon Chemicals) | |
| 12-hydroxystearic acid | 0.4 |
| Merquart 3300 | 0.4 |
| (10% aqueous solution of polyquaternium-39 from Nalco) | |
| Cationized locust bean gum | 0.2 |
| Triglyceride 2-ethylhexanoate | 0.2 |
| Talc | 1.0 |
| Sodium benzoate | 0.2 |
| Dimethylsilicone | 0.2 |
| (4,000 cs-silicone made into a particle size of 200 nm) | |
| Perfume | 0.1 |
| Ion-exchanged water | Balance |

| Recipe example 5 (Face cleaning agent) | |
|---|---|
| Sodium cocoyl taurate | 13.0 mass % |
| Sodium myristoyl L-glutamate | 5.0 |
| PEG 1000 | 10.0 |
| Glycerin | 10.0 |
| Sodium sulfate | 4.0 |
| Na L-glutamate | 0. 6 |
| L-glutamic acid | 2. 4 |
| Sodium N-methyltaurate | 3.0 |
| Nikkol HCO-60 | 1.0 |
| (POE hydrogenated castor oil (60EO) from Nihon Chemicals) | |
| Stearic acid | 0. 2 |
| Carboxyvinyl polymer | 0. 2 |
| Batyl alcohol | 0. 2 |
| Ethylene glycol distearate | 0.2 |
| Talc | 1. 0 |
| Na benzoate | 0.2 |
| Pentaerysrityl tetraoctanoate | 0. 1 |
| Perfume | 0.1 |
| Ion-exchanged water | Balance |

[B: Invention defined in claims 8-14]

The second portion of the present invention is described in detail below by referring to Examples, but the present invention is not limited to these Examples. The blend ratios are all in mass % units.

[(a)-(d) ingredient blend system]:

"Examples 1-6, Comparative examples 1-5"

Cleaning agent compositions (samples) consisting of the compositions (mass %) shown in the following Tables 1b and 2b were prepared. Preparation was conducted by stirring and dissolving the ingredients at 75°C and cooling them down to 25°C at a constant rate. The obtained samples were evaluated for stability, foaming, and usability (refreshing sensation).

The results are shown in Table 1b and 2b.

Stability, foaming, and usability (refreshing sensation) were evaluated by using the following test methods and evaluation methods.

[Stability]

After the samples were prepared, they were stored in a 40°C thermostatic bath for two months and then the external appearance was evaluated by using the following criteria. The viscosity was measured by using a B-type viscometer.

(Evaluation)

○: Even at 40°C, the viscosity is maintained at 3,000 mPa·s or higher and no separation was observed.
Δ: At 40°C, the viscosity is 3,000 mPa·s or less, but no separation is observed.
✕: Separation occurs at 40°C and a clear layer is observed.

[Foaming]

4 g of each sample was added to 400 mL of distilled water containing 100 ppm calcium chloride (hard water, 30°C); this was then put into a 2,500 mL glass container and stirred for 10 seconds at 4,500 cycles with the mixer method, after which the amount of foam was measured. The evaluation was conducted by using the following criteria.

(Evaluation)

○: Good foaming (the amount of foam is 1600 mL or more)
Δ: Moderate foaming (the amount of foam is 1100 mL or more and less than 1600 mL)
✕: Poor foaming (the amount of foam is less than 1100 mL)

[Refreshing sensation at the time of rinsing]

A panel of 30 specialists used 1 g of the sample to wash their faces in a normal way; the sensory evaluation of the refreshing sensation at the time of rinsing (no sliminess) was conducted using the following criteria.

(Evaluation)

◎: 30 (all) reported a lack of sliminess at the time of rinsing.
○: 25-29 reported a lack of sliminess at the time of rinsing.
Δ: 15-24 reported a lack of sliminess at the time of rinsing.
✕: 14 or less reported a lack of sliminess at the time of rinsing.

A differential scanning calorimeter (DSC) was used to measure the endothermic peak of the sample for water when raising the temperature; the melting point of the sample was defined as the peak top.

Table 1b

| Ingredients | Comparative example 1 | Comparative example 2 | Comparative example 3 | Comparative example 4 | Example 1 | Example 2 |
|---|---|---|---|---|---|---|
| Sodium lauroyl methyltaurate | 15 | 15 | 15 | - | 15 | 15 |

Table 1b   (continued)

| Ingredients | Comparative example 1 | Comparative example 2 | Comparative example 3 | Comparative example 4 | Example 1 | Example 2 |
|---|---|---|---|---|---|---|
| Sodium myristoyl methyltaurate | 5 | 5 | 5 | - | 5 | 5 |
| Sodium myristoyl glutamate | - | - | - | 20 | - | - |
| Sodium chloride | - | - | 5 | - | 5 | 10 |
| Glycerin | - | 20 | - | - | 20 | 2 |
| Ion-exchanged water | Balance | Balance | Balance | Balance | Balance | Balance |
| Melting point (°C) | 10 | 22 | 35 | 45 | 45 | 49 |
| Stability (40°C, 2 months) | × | × | × | ○ | ○ | ○ |
| Foaming | ○ | ○ | ○ | × | ○ | ○ |
| Sensation during use (refreshing sensation) | × | Δ | Δ | ○ | ○ | ◎ |

The (a) ingredient consisting of a mixed system of two anionic surfactants (sodium lauroyl methyltaurate and sodium myristoyl methyltaurate) has a Kraft point of approximately 10°C. As clearly shown in the results in Table 1b, a cleaning agent composition that is in the paste/solid form and exhibits superior stability and good foaming and refreshing sensation can be obtained from this (a) ingredient system by adding sodium chloride as the (b) ingredient that has the same type of metal ion as the counter ion of the aforementioned (a) ingredient, glycerin as the (c) ingredient, and water as the (d) ingredient to increase the melting point to 40°C or higher.

In Comparative example 4, a system consisting solely of an anionic surfactant having a Kraft point of approximately 45°C (sodium myristoyl glutamate) was used, but foaming was poor.

Table 2b

| Ingredients | Example 3 | Example 4 | Example 5 | Example 6 | Comparative example 5 |
|---|---|---|---|---|---|
| Sodium lauroyl methyltaurate | 10 | 10 | 10 | 10 | 10 |
| Calcium chloride | - | 1.7 | 5 | 8.3 | 11.7 |
| Sodium chloride | 8.8 | 7.9 | 6.1 | 4.4 | 2.6 |
| Sorbitol | 10 | 10 | 10 | 10 | 10 |
| Ion-exchanged water | Balance | Balance | Balance | Balance | Balance |
| (b) Ingredient moles | 0. 15 | 0. 15 | 0. 15 | 0. 15 | 0. 15 |
| Calcium chloride : Sodium chloride (molar ratio) | 0: 10 | 1: 9 | 3: 7 | 5: 5 | 7: 3 |
| Melting point (°C) | 50 | 48 | 46 | 44 | 38 |
| Stability (40°C, 2 months) | ○ | ○ | ○ | ○ | × |
| Foaming | ○ | ○ | ○ | ○ | Δ |

Table 2b   (continued)

| Ingredients | Example 3 | Example 4 | Example 5 | Example 6 | Comparative example 5 |
|---|---|---|---|---|---|
| Sensation during use (refreshing sensation) | ○ | ○ | ◎ | ◎ | ◎ |

A mixed system of calcium chloride, which is a salt of a different metal than the counter ion of the (a) ingredient, and sodium chloride, which is a salt of the same metal as the counter ion of the (a) ingredient, was used for the (b) ingredient. The number of moles of the (b) ingredient was set at a constant value of 0.15 and the ratio (molar ratio) of the aforementioned two inorganic salts was varied. When the ratio (molar ratio) of the calcium chloride versus sodium chloride becomes higher, the refreshing sensation improves but the melting point of the cleaning agent composition tends to decrease.

The Kraft point of sodium lauroyl methyltaurate by itself is 0°C or lower.

Example 7 (Face cleaning agent)

| (Ingredients) | (mass %) |
|---|---|
| Sodium lauroyl ether carboxylate (Kraft point 5°C ) | 21 |
| Magnesium chloride | 4 |
| Sodium chloride | 8 |
| Glycerin | 15 |
| pH adjustment agent | Appropriate amount |
| Preservative | Appropriate amount |
| Perfume | Appropriate amount |
| Ion-exchanged water | Balance |

(Preparation method and evaluation)

A cleaning agent composition consisting of the aforementioned composition was prepared with a conventional method. The obtained cleaning agent composition had a melting point of 53°C, assumed a paste/solid form and exhibited superior stability (40°C) and good foaming and refreshing sensation during use.

(Example 8: Hair shampoo)

| (Ingredients) | (mass %) |
|---|---|
| Sodium polyoxyethylene (2 moles) laurylsulfate (Kraft point: 0°C or lower) | 5 |
| Sodium myristoyl methyltaurate (Kraft Point: 23°C) | 15 |
| Sodium citrate | 3 |
| Citric acid | 2 |
| Sorbitol | 30 |
| Preservative | Appropriate amount |
| Perfume | Appropriate amount |
| Ion-exchanged water | Balance |

(Preparation method and evaluation)

A cleaning agent composition consisting of the aforementioned composition was prepared with a conventional method. The obtained cleaning agent composition had a melting point of 44°C, assumed a paste/solid form and exhibited superior stability (40°C) and good foaming and refreshing sensation during use.

(Example 9: Body shampoo)

| (Ingredients) | (mass %) |
|---|---|
| Sodium dodecane-1,2-diol acetate ether (Kraft point: 0°C or lower) | 25 |
| Sodium chloride | 9 |
| Calcium chloride | 3 |
| Sodium citrate | 1.6 |
| Citric acid | 1.4 |
| Glycerin | 10 |
| Preservative | Appropriate amount |
| Perfume | Appropriate amount |
| Ion-exchanged water | Balance |

(Preparation method and evaluation)

A cleaning agent composition consisting of the aforementioned composition was prepared with a conventional method. The obtained cleaning agent composition had a melting point of 43°C, assumed a paste/solid form and exhibited superior stability (40°C) and good foaming and refreshing sensation during use.

[(a)-(d) ingredient + (e) ingredient blend system]:

(Examples I-III, Comparative examples I-V)

Cleaning agent compositions (samples) consisting of the compositions (mass %) shown in the following Tables 3b and 4b were prepared. Preparation was conducted by stirring and dissolving the ingredients at 75°C and cooling them down to 25°C at a constant rate. The obtained samples were evaluated for stability, foaming, usability (refreshing sensation), external appearance stability (45°C), tingling sensation during use, and foam quality. The results are shown in Table 3b and 4b.
Stability, foaming, and usability (refreshing sensation) were evaluated by using the aforementioned methods. The melting point was also measured by using the aforementioned method. The external 1 appearance (45°C), tingling sensation during use, and foam quality were evaluated by using the following test methods and evaluation methods.

[External appearance stability]

After the sample was prepared, a prescribed amount of it was put into a glass bottle and stored in a 45°C thermostatic bath for one week, after which the external appearance was evaluated based on the following criteria.

(Evaluation)

⊚ : The whole sample is homogeneously white and turbid; no clear portion is observed.
○ : A clear portion is observed in less than 5% of the whole sample.
○Δ : A clear portion is observed in 5% or more and less than 10% of the whole sample.
Δ : A clear portion is observed in 10% or more and less than 40% of the whole sample.
✕ : A clear portion is observed in 40% or more of the whole sample.

[Tingling sensation during use]

A panel of 20 specialists applied 1 g of the sample directly on the face and washed the face; the sensory evaluation of the tingling sensation at that time was conducted using the following criteria.

(Evaluation)

◎ : 18 or ore reported there was no tingling sensation during use.
○ : 15-17 reported there was no tingling sensation during use.
○Δ : 12-14 reported there was no tingling sensation during use.
Δ : 9-11 reported there was no tingling sensation during use.
× : 8 or less reported there was no tingling sensation during use.

[Foam quality (creaminess)]

A panel of 20 specialists used 1 g of the sample to wash their faces in a normal way; the sensory evaluation of the foam quality (creaminess) was conducted using the following criteria.

(Evaluation)

◎ : 16 or more reported creaminess.
○ : 13-15 reported creaminess.
○Δ : 10-12 reported creaminess.
Δ : 7-9 reported creaminess.
× : 6 or less reported creaminess.

Table 3b

| Ingredients | Comparative example I | Comparative example II | Comparative example III | Example I |
|---|---|---|---|---|
| Sodium lauroyl methyltaurate | 15 | 15 | - | - |
| Sodium myristoyl methyltaurate | 5 | 5 | - | - |
| Sodium methyl cocoyl taurate | - | - | 20 | 20 |
| Sodium chloride | 5 | 10 | 5.5 | 5.5 |
| Sodium citrate | - | - | 0.6 | 0.6 |
| Citric acid | - | - | 0.4 | 0.4 |
| Glycerin | 20 | 2 | 20 | 20 |
| Sorbitol | - | - | - | - |
| Taurine | - | - | - | 2 |
| Sodium N-methyltaurate | - | - | - | - |
| Sodium benzoate | - | - | 0.3 | 0.3 |
| Ion-exchanged water | Balance | Balance | Balance | Balance |
| Melting point (°C) | 45 | 49 | 50.8 | 52.9 |
| Stability (40°C, 2 months) | ○ | ○ | ○ | ○ |
| Foaming | ○ | ○ | ○ | ○ |

Table 3b (continued)

| Ingredients | Comparative example I | Comparative example II | Comparative example III | Example I |
|---|---|---|---|---|
| Sensation during use (refreshing sensation) | ○ | ◎ | ○ | ○ |
| External appearance stability (45°C, 1 week) | × | ○Δ | ○Δ | ◎ |
| Tingling sensation during use | ○Δ | Δ | Δ | ○ |
| Foam quality (creaminess) | × | Δ | Δ | ○ |

Table 4b

| Ingredients | Example II | Comparative example IV | Comparative example V | Example III |
|---|---|---|---|---|
| Sodium lauroyl methyltaurate | - | - | 10 | 10 |
| Sodium myristoyl methyltaurate | - | - | - | - |
| Sodium methyl cocoyl taurate | 20 | 20 | - | - |
| Sodium chloride | 5.5 | 5.5 | 8.8 | 6.8 |
| Sodium citrate | 0.6 | 2.6 | - | 0. 6 |
| Citric acid | 1. 6 | 0.4 | - | 2.5 |
| Glycerin | 20 | 20 | - | - |
| Sorbitol | - | - | 10 | 10 |
| Taurine | - | - | - | - |
| Sodium N-methyltaurate | 2.5 | - | - | 4 |
| Sodium benzoate | 0. 3 | 0. 3 | - | 0. 3 |
| Ion-exchanged water | Balance | Balance | Balance | Balance |
| Melting point (°C) | 55.7 | 54 | 50 | 55.7 |
| Stability (40°C, 2 months) | ○ | ○ | ○ | ○ |
| Foaming | ○ | ○ | ○ | ○ |
| Sensation during use (refreshing sensation) | ○ | ○ | ○ | ○ |
| External appearance stability (45°C, 1 week) | ◎ | ◎ | Δ | ◎ |
| Tingling sensation during use | ○ | Δ | Δ | ○ |
| Foam quality (creaminess) | ○ | Δ | × | ○ |

In Tables 3b and 4b, Comparative examples I-V were (a)-(d) ingredient system samples within the range of the present invention, all of which were confirmed to have superior stability, foaming, and usability (refreshing sensation); however they did not contain the (e) ingredient and there was no effect in terms of the tingling sensation during use, foam quality (creaminess), etc. In contrast, Examples I-III, which contain taurines as the (e) ingredient in addition to the (a)-(d) ingredients, were confirmed to be superior in terms of the external appearance stability (45°C), lack of a tingling sensation during use, and foam quality (creaminess), in addition to being superior in terms of stability, foaming, and usability (refreshing sensation).

(Examples IV-X, Comparative examples VI-IX)

Cleaning agent compositions (samples) consisting of the compositions (mass %) shown in the following Tables 5b-7b were prepared. Preparation was conducted by stirring and dissolving the ingredients at 75°C and cooling them down to 25°C at a constant rate. The obtained samples were evaluated for stability, foaming, usability (refreshing sensation), external appearance stability (45°C), tingling sensation during use, and foam quality by using the aforementioned evaluation criteria. The results are shown in Table 5b-7b.

Table 5b

| Ingredients | Comparative example VI | Comparative example VI | Example I IV | Example V |
|---|---|---|---|---|
| Sod i un methyl cocoyl taurate | 20 | 20 | 20 | 20 |
| Sodium chloride | 5.5 | 5.5 | 5.5 | 5.5 |
| Sodium citrate | 0.6 | 0.6 | 0.6 | 0.6 |
| Citric acid | 0.4 | 0.4 | 0.4 | 0.4 |
| Glycerin | 20 | 20 | 20 | 20 |
| Sorbitol | - | - | 3 | - |
| POE (8) glycerin monoisostearate (HLB=9) | - | 2 | - | - |
| POE (15) glycerin monoisostearate (HLB=12) | - | - | 2 | - |
| POE (25) glycerin monoisostearate (HLB=15) | - | - | - | 2 |
| POE (30) glycerin monoisostearate (HLB=18) | - | - | - | - |
| POE (60) glycerin monoisostearate (HLB=19) | - | - | - | - |
| POE (90) glycerin monoisostearate (HLB=22) | - | - | - | - |
| POE (25) octyldodecyl ether (HLB=14) | - | - | - | - |
| POE (60) hydrogenated castor oil (HLB=14) | - | - | - | - |
| POE (1) · POP (8) cetyl ether (HLB=9.5) | - | - | - | - |
| Propylene glycol laurate (HLB=4.5) | - | - | - | - |
| Sodium benzoate | 0. 3 | 0.3 | 0.3 | 0.3 |
| Ion-exchanged water | Balance | Balance | Balance | Balance |
| Melting point (°C) | 50.8 | 50.6 | 51.9 | 51.3 |
| Stability (40°C, 2 months) | ○ | ○ | ○ | ○ |
| Foaming | ○ | ○ | ○ | ○ |
| Sensation during use (refreshing sensation) | ○ | ○ | ○ | ○ |
| External appearance stability (45°C, 1 week) | ○Δ | Δ | ○ | ○ |

Table 5b   (continued)

| Ingredients | Comparative example VI | Comparative example VI | Example I IV | Example V |
|---|---|---|---|---|
| Tingling sensation during use | Δ | ○Δ | ○ | ○ |
| Foam quality (creaminess) | Δ | ○Δ | ○ | ○ |

Table 6b

| Ingredients | Example VI | Example VII | Example VIII | Example IX |
|---|---|---|---|---|
| Sodium methyl cocoyl taurate | 20 | 20 | 20 | 20 |
| Sodium chloride | 5.5 | 5.5 | 5.5 | 5.5 |
| Sodium citrate | 0.6 | 0.6 | 0.6 | 0.6 |
| Citric acid | 0.4 | 0.4 | 0.4 | 0.4 |
| Glycerin | 20 | 20 | 20 | 20 |
| Sorbitol | - | - | - | - |
| POE (8) glycerin monoisostearate (HLB=9) | - | - | - | - |
| POE (15) glycerin monoisostearate (HLB=9) | - | - | - | - |
| POE (25) glycerin monoisostearate (HLB=15) | - | - | - | - |
| POE (30) glycerin monoisostearate (HLB=18) | 2 | - | - | - |
| POE (60) glycerin monoisostearate (HLB=19) | - | 2 | - | - |
| POE (90) glycerin monoisostearate (HLB=22) | - | - | 2 | - |
| POE (25) octyldodecyl ether (HLB=14) | - | - | - | 2 |
| POE (60) hydrogenated castor oil (HLB=14) | - | - | - | - |
| POE (1) · POP (8) cetyl ether (HLB=9.5) | - | - | - | - |
| Propylene glycol laurate (HLB=4.5) | - | - | - | - |
| Sodium benzoate | 0.3 | 0.3 | 0.3 | 0.3 |
| Ion-exchanged water | Balance | Balance | Balance | Balance |
| Melting point (°C) | 52.0 | 51.7 | 51.5 | 52.8 |
| Stab ility (40°C, 2 months) | ○ | ○ | ○ | ○ |
| Foaming | ○ | ○ | ○ | ○ |
| Sensation during use (refreshing sensation) | ○ | ○ | ○ | ○ |
| External appearance stability (45°C, 1 week) | ○ | ○ | ○ | ◎ |
| Tingling sensation during use | ○ | ○ | ○ | ○ |
| Foam quality (creaminess) | ○ | ○Δ | ○Δ | ○ |

Table 7b

| Ingredients | Example X | Comparative example VIII | Comparative example IX |
|---|---|---|---|
| Sodium methyl cocoyl taurate | 20 | 20 | 20 |
| Sodium chloride | 5.5 | 5.5 | 5.5 |
| Sodium citrate | 0.6 6 | 0.6 | 0.6 6 |
| Citric acid | 0.4 | 0.4 | 0.4 |

Table 7b   (continued)

| Ingredients | Example X | Comparative example VIII | Comparative example IX |
|---|---|---|---|
| Glycerin | 20 | 20 | 20 |
| Sorbitol | 3 | - | - |
| POE (8) glycerin monoisostearate (HLB=9) | - | - | - |
| POE (15) glycerin monoisostearate (HLB=9) | - | - | - |
| POE (25) glycerin monoisostearate (HLB=15) | - | - | - |
| POE (30) glycerin monoisostearate (HLB=18) | - | - | - |
| POE (60) glycerin monoisostearate (HLB=19) | - | - | - |
| POE (90) glycerin monoisostearate (HLB=22) | - | - | - |
| POE (25) octyldodecyl ether (HLB=14) | - | - | - |
| POE (60) hydrogenated castor oil (HLB=14) | 2 | - | - |
| POE (1) · POP (8) cetyl ether (HLB=9.5) | - | 2 | - |
| Propylene glycol laurate (HLB=4.5) | - | - | 2 |
| Sodium benzoate | 0.3 | 0.3 | 0.3 |
| Ion-exchanged water | Balance | Balance | Balance |
| Melting point (°C) | 52.0 | 49.5 | 47.1 |
| Stability (40°C, 2 months) | ○ | ○ | ○ |
| Foaming | ○ | ○ | ○ |
| Sensation during use (refreshing sensation) | ○ | ○ | ○ |
| External appearance stability (45°C, 1 week) | ○ | ○ | ○ |
| Tingling sensation during use | ○ | ○Δ | Δ |
| Foam quality (creaminess) | ○ | ○Δ | ○Δ |

As clearly shown in the results in Tables 5b-7b, all the samples containing the (a)-(d) ingredients exhibited superior stability, foaming, and usability (refreshing sensation); however, those without the (e) ingredient did not have the effects in terms of the tingling sensation during use, foam quality, etc. In contrast, the samples that contained a nonionic surfactant having a HLB of 10 or more as the (e) ingredient in addition to the (a)-(d) ingredients were confirmed to be superior in terms of the external appearance stability (45°C), lack of a tingling sensation during use, and foam quality (creaminess), in addition to being superior in terms of stability, foaming, and usability (refreshing sensation).

(Examples XI-XIV)

Cleaning agent compositions (samples) consisting of the compositions (mass %) shown in the following Table 8b were prepared. Preparation was conducted by stirring and dissolving the ingredients at 75°C and cooling them down to 25°C at a constant rate. The obtained samples were evaluated for stability, foaming, usability (refreshing sensation), external appearance stability (45°C), tingling sensation during use, and foam quality by using the aforementioned evaluation criteria. The results are shown in Table 8b.

Table 8b

| Ingredients | Example XI | Example XII | Example XIII | Example XIV |
|---|---|---|---|---|
| Sodium methyl cocoyl taurate | 20 | 20 | 20 | 20 |
| Sodium chloride | 5.5 | 5.5 | 5.5 | 5.5 |
| Sodium citrate | 0.6 | 0.6 | 0.6 | 0.6 |
| Citric acid | 0.4 | 0.4 | 0.4 | 1. 6 |
| Glycerin | 20 | 20 | 15 | 15 |
| Sorbitol | - | - | 5 | 5 |
| Taurine | 2 | - | 2 | - |
| Sodium N-methyltaurate | - | - | - | 2.5 |
| POE (25) glycer in monoisostearate (HLB=15) | - | 2 | 1.5 | - |
| POE (25) octyldodecyl ether (HLB=14) | - | - | - | 1.5 |
| Sodium benzoate | 0.3 | 0.3 | 0.3 | 0.3 |
| Ion-exchanged water | Balance | Balance | Balance | Balance |
| Melting point (°C) | 52.9 | 51.3 | 52.0 | 55.8 |
| Stability (40°C, 2 months) | ○ | ○ | ○ | ○ |
| Foaming | ○ | ○ | ○ | ○ |
| Sensation during use (refreshing sensation) | ○ | ○ | ○ | ○ |
| External appearance stability (45°C, 1 week) | ◎ | ○ | ○ | ◎ |
| Tingling sensation during use | ○ | ○ | ◎ | ◎ |
| Foam quality (creaminess) | ○ | ○ | ◎ | ◎ |

As clearly shown in Table 8b, the samples that contained taurines and a nonionic surfactant having a HLB of 10 or more as the (e) ingredient in addition to the (a)-(d) ingredients were confirmed to be excellent in terms of the external appearance stability (45°C), lack of a tingling sensation during use, and foam quality (creaminess), in addition to being superior in terms of stability, foaming, and usability

(refreshing sensation).

(Example XV: Body shampoo)

| (Ingredients) | (mass %) |
|---|---|
| Sodium methyl cocoyl taurate (Kraft point: 12°C) | 20 |
| Glycerin | 20 |
| Sorbitol | 5 |
| Sodium chloride | 5 |
| Sodium L-glutamate | 0.4 |
| L-glutamic acid | 0.8 |
| Taurine | 4 |
| POE (60) glycerin monoisostearate (HLB=19) | 0. 5 |
| POE (25) octyldodecyl ether (HLB=14) | 1 |
| Behenic acid | 1. 5 |
| 2-octyldodecanol | 0. 1 |
| Cationized polymer ("Merquart 100" from Nalco: 40% aqueous solution of polyquaternium-6) | 0.3 |

(continued)

| (Ingredients) | (mass %) |
|---|---|
| Silica (spherical, average particle size 2 micrometers) | 0.5 |
| Trehalose | 0. 2 |
| Phenoxy ethanol | 0. 1 |
| Perfume | 0. 1 |
| Ion-exchanged water | Balance |

(Example XVI: Face cleaning agent)

| (Ingredients) | (mass %) |
|---|---|
| Sodium methyl cocoyl taurate (Kraft point: 23°C) | 10 |
| Glycerin | 15 |
| Sorbitol | 8 |
| Sodium chloride | 4 |
| Sodium tartarate | 0.6 |
| Tartaric acid | 0.4 |
| Taurine | 4 |
| POE (20) glycerin monoisostearate (HLB=14) | 2 |
| POE (60) hydrogenated castor oil (HLB=14) | 1 |
| Monoglyceride stearate | 0.5 |
| Cationized polymer ("Merquart 550" from Nalco: 10% aqueous solution of polyquaternium-7) | 1 |
| Mica | 0. 5 |
| Methylparaben | 0. 1 |
| L-hydroxyproline | 0. 1 |
| Perfume | 0. 1 |
| Ion-exchanged water | Balance |

(Example XVII: Hair shampoo)

| (Ingredients) | (mass %) |
|---|---|
| Potassium methyl cocoyl taurate (Kraft point: 34°C) | 15 |
| Glycerin | 10 |
| Sorbitol | 10 |
| Potassium chloride | 4 |
| Sodium citrate | 0.6 |
| Citric acid | 2.4 |
| Sodium N-methyltaurate | 3 |
| POE (25)·POP (30) copolymer ("Pluaronic L-64") | 0.5 |
| 12-hydroxystearic acid | 0.4 |
| Cationized cellulose ("Polymer JR" from TOHO Chemical Industry) | 0.4 |
| Cationized guar gum (Jaguar C-13S) | 0.4 |
| Dimethylsilicone (10,000 cs-silicone made into a particle size of 30 nm) | 0.2 |
| Talc | 1 |
| Zanthoxylum extract | 1 |
| Sodium benzoate | 0.2 |
| Perfume | 0. 1 |

(continued)

| (Ingredients) | (mass %) |
|---|---|
| Ion-exchanged water | Balance |

(Example XIII: Face cleaning agent)

| (Ingredients) | (mass %) |
|---|---|
| Sodium methyl cocoyl taurate (Kraft point: 12°C) | 10 |
| Sodium cocoyl isethionate | 10 |
| Glycerin | 15 |
| Sorbitol | 10 |
| Sodium sulfate | 4 |
| Sodium malate | 0.6 |
| Malic acid | 2.4 |
| Sodium N-methyltaurate | 3 |
| POE (60) hydrogenated castor oil (HLB=14) | 1 |
| 12-hydroxystearic acid | 0.4 |
| Ampholytic polymer ("Merquart 3300" from Nalco: 10% aqueous solution of polyquaternium-39) | 0.4 |
| Cationized locust bean gum | 0.2 |
| Triglyceride 2-ethylhexanoate | 0.2 |
| Talc | 0.2 |
| Sodium benzoate | 0.2 |
| Dimethylsilicone (4,000 cs-silicone made into a particle size of 200 nm) | 0.2 |
| Perfume | 0. 1 |
| Ion-exchanged water | Balance |

(Example XIX: Face cleaning agent)

| (Ingredients) | (mass %) |
|---|---|
| Sodium methyl cocoyl taurate (Kraft point: 23°C) | 13 |
| Sodium stearoyl methyltaurate | 1 |
| Sodium myristoyl L-glutamate | 5 |
| Glycerin | 10 |
| Sorbitol | 10 |
| Sodium sulfate | 4 |
| Sodium L-glutamate | 0.6 |
| L-glutamic acid | 2.4 |
| Sodium N-methyltaurate | 3 |
| POE (60) hydrogenated castor oil (HLB=14) | 1 |
| Stearic acid | 0.2 |
| Carboxyvinyl polymer | 0.2 |
| Batyl alcohol | 0.2 |
| Ethylene glycol distearate | 0.2 |
| Talc | 1 |
| Sodium benzoate | 0.2 |
| Pentaerysrityl tetraoctanoate | 0.1 |
| Perfume | 0. 1 |

(continued)

| (Ingredients) | (mass %) |
|---|---|
| Ion-exchanged water | Balance |

INDUSTRIAL APPLICABILITY

As described in detail thus far, the first portion of the present invention provides a cleaning agent composition that assumes a stable paste or solid form over a wide temperature range and exhibits good foaming as well as a sensation during use that is free of a tingling sensation on the skin.

The second portion of the present invention provides a cleaning agent composition that assumes a stable paste or solid form over a wide temperature range and exhibits good foaming as well as a sensation during use that is free of sliminess at the time of rinsing. Also, the present invention provides a cleaning agent composition in a paste/solid form that gives a refreshing tactile sensation, exhibits a satisfactory foaming performance and higher stability, and also gives no tingling sensation during use and provides creamy foam quality.

**Claims**

1. A paste or solid cleaning agent composition comprising (a) 5-50 mass % of an acyl salt anionic surfactant represented by the following general formula (1), (b) one, two or more chosen from inorganic salts, organic acids, and organic salts, (c) polyethylene glycol, and (d) water wherein the total electrolyte molar concentration is 1.8 mol/kg or more.

$$R^1CO\text{-}NR^2CH_2CH_2SO_3X \qquad (1)$$

(In this formula, $R^1$ denotes a hydrocarbon group having 10-24 carbons, $R^2$ denotes a hydrogen atom or methyl group, and X denotes an alkali metal, alkali earth metal, ammonium, or organic amine. )

2. The cleaning agent composition of claim 1 wherein $R^1$ of the (a) ingredient comprises a lauric acid residue and/or myristic acid residue.

3. The cleaning agent composition of claim 1 wherein the organic acid and organic salt of the (b) ingredient is one, two or more chosen from citric acid, succinic acid, lactic acid, malic acid, glycolic acid, tartaric acid, and acidic amino acids, as well as their salts.

4. The cleaning agent composition of claim 1 wherein the molecular weight of the (c) ingredient is 200-1,000.

5. The cleaning agent composition of claim 1 that further comprises (e) a nonionic surfactant.

6. The cleaning agent composition of claim 5 wherein the (e) ingredient is a polyoxyethylene glycerin monoisostearate having a HLB of 10-15.

7. The cleaning agent composition of claim 1 that further comprises (f) one, two or more chosen from taurine, N-methyltaurine, and N,N-dimethyltaurine.

8. A paste or solid cleaning agent composition having a system melting point of 40°C or higher, comprising (a) an anionic surfactant having a Kraft point of 40°C or lower, (b) one, two, or more chosen from inorganic salts and organic salts, (c) trihydric or higher polyol, and (d) water.

9. The cleaning agent composition of claim 8 wherein ingredient (a) is one, two, or more chosen from long chain acyl taurine salts represented by the following general formula (I).

$$R_1CO\text{-}NR_2\text{-}CH_2CH_2SO_3M \qquad (I)$$

(In this formula, $R_1$ denotes a saturated or unsaturated hydrocarbon group with an average number of carbon atoms of 7-19; $R_2$ denotes an alkyl group with an average number of carbon atoms of 1-3; and M denotes an alkali metal, alkali earth metal, ammonium, or organic amine or derivative.)

10. The cleaning agent composition of claim 8 or 9 wherein at least one of the constituents of the (b) ingredient is the same type of metal ion salt as the counter ion of the (a) ingredient.

11. The cleaning agent composition of claim 10 wherein, in the (b) ingredient, the molar ratio of the same type of metal ion salt as the counter ion of the (a) ingredient is one or more to the metal ion salt of types other than the counter ion of the (a) ingredient.

12. The cleaning agent composition of any one of claims 8-11 that further comprises (e) one, two or more chosen from taurines and nonionic surfactants having a HLB of 10 or more.

13. The cleaning agent composition of claim 12 wherein the nonionic surfactant having a HLB of 10 or more, used for the (e) ingredient, is one, two or more chosen from POE (=polyoxyethylene) glycerin monoisostearate, POE dialkyl ether, and POE hydrogenated castor oil, as well as derivatives thereof.

14. The cleaning agent composition of claim 12 or 13 wherein the melting point of the system is 45°C or higher.

**EP 1 516 914 A1**

**INTERNATIONAL SEARCH REPORT**

| International application No. |
| --- |
| PCT/MX 02/00058 |

A. CLASSIFICATION OF SUBJECT MATTER

**IPC7:** A61K 33/06, C07C 59/08, 105, A61P 19/00, A61K 9/46

According to International Patent Classification (IPC) or to both national classification and IPC

B. FIELDS SEARCHED

Minimum documentation searched (classification system followed by classification symbols)

**IPC7:**

Documentation searched other than minimum documentation to the extent that such documents are included in the fields searched

Electronic data base consulted during the international search (name of data base and, where practicable, search terms used)

STN, EPODOC, OEPMPAT

C. DOCUMENTS CONSIDERED TO BE RELEVANT

| Category* | Citation of document, with indication, where appropriate, of the relevant passages | Relevant to claim No. |
| --- | --- | --- |
| X <br> A | EP 885568 A(DIEPHARMEX) 23.12.1998; <br> column 1, 2, 5 and 6 | 3-7, 9 <br> 8 |
| X <br> A | ES 8609192 A (CRESPO Y HEREDEROS S.L.) 16.12.1986; **pages** 2, line 11 - **page** 4, line 10. | 3, 4 <br> 1, 2 |
| A | US 2813892 A (CHARLES L. MEHLTRETTER) 19.11.1957; column 1, line 21-36; column 2, **example** | 1, 2 |
| X | FR 2601249 A (SANDOZ LAB.) 15.01.1988; **pages** 1,2 and 5. | 3-7, 9 |

| ☐ Further documents are listed in the continuation of Box C. | **☒** See patent family annex. |
| --- | --- |

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **8 october (08.10.2002)** | **17 october (17.10.2002)** |

| Name and mailing address of the ISA/ <br><br> **S.P.T.O** | Authorized officer <br><br> Alicia COLOMER NIEVES |
| --- | --- |
| Facsimile No. | Telephone No. |

Form PCT/ISA/210 (second sheet) (July 1992)

31

## INTERNATIONAL SEARCH REPORT
Information on patent family members

| International Application No |
| --- |
| PCT/MX 02/00058 |

| Patent document cited in search report | Publication date | Patent familiy member(s) | Publication date |
| --- | --- | --- | --- |
| Documento de patente citado en el informe de búsqueda | Fecha de publicación | Miembro(s) de la familia de patentes | Fecha de publicación |
| EP 885568 A | 23.12.1998 | FR 2764477 AB | 18.12.1998 |
| ES 8609192 A | 16.12.1986 | NONE | |
| US 2813892 A | 19.11.1957 | NONE | |
| FR 2601249 A | 15.01.1988 | NONE | |

Form PCT/ISA/210 (patent family annex) (July 1992)

32